(19) Europäisches Patentamt
European Patent Office
Office européen des brevets

(11) Publication number: 0 096 640 B1

# (12) EUROPEAN PATENT SPECIFICATION

(45) Date of publication of patent specification: 26.02.86

(51) Int. Cl.$^4$: C 07 C 120/00, C 07 C 121/75, C 07 C 51/08

(21) Application number: 83401146.2

(22) Date of filing: 06.06.83

## (54) Preparation of 4-(alpha-alkyl-alpha-cyano-methyl)-2,6-di-substituted phenols.

(30) Priority: 07.06.82 US 385609

(43) Date of publication of application:
21.12.83 Bulletin 83/51

(45) Publication of the grant of the patent:
26.02.86 Bulletin 86/09

(84) Designated Contracting States:
BE CH DE FR GB LI

(56) References cited:
EP-A-0 048 841
US-A-3 496 211

Chemical Abstracts, vol. 96, no. 25, 21 June 1982, Columbus, Ohio, USA
Chemical Abstracts, vol. 58, no. 10, 13 May 1963, Columbus, Ohio, USA, J.N. Coker et al. "The cyanomethylation of indole", column 10152de

(73) Proprietor: ETHYL CORPORATION
Ethyl Tower 451 Florida Boulevard
Baton Rouge Louisiana 70801 (US)

(72) Inventor: Everly, Charles R.
1431 Munal Drive
Baton Rouge Louisiana 70816 (US)
Inventor: Robinson, Gene C.
1064 North Leighton Drive
Baton Rouge Louisiana 70806 (US)

(74) Representative: Rinuy, Guy et al
14, Avenue de la Grande Armée
F-75017 Paris (FR)

(56) References cited:

Chemical Abstracts, vol. 65, no. 7, 26 September 1966, Columbus, Ohio, USA, A.A. VOLOD'KIN et al. "Reaction of alpha-alkyl-4-hydroxy-3,5-di-tert-butylbenzyl halides with some nucleophilic reagents"
Kirk-Othmer, Encyclopedia of chemical technology, 3rd ed., vol. 2 (1978), 85-86

EP 0 096 640 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

This invention relates to a novel process for the preparation of 4 - (α - alkyl - α - cyano - methyl)2,6 - di - substituted phenols having the formula:

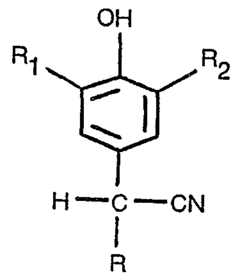

wherein R is hydrogen, methyl, ethyl, *n*-propyl, or isopropyl and $R_1$ and $R_2$ are the same or different monovalent substituent selected from the group consisting of alkyl, aralkyl and cyclic alkyl radicals. Further, this invention relates to the use of 4 - (α - alkyl - α - cyano - methyl)2,6 - di - substituted phenols as intermediates in a reaction sequence in which α - alkyl - 4 - hydroxyphenylacetic acids are produced which in turn may be used as reaction intermediates in the preparation of insecticides of m-phenoxybenzyl and α - cyano - m - phenoxybenzyl esters.

Meta-phenoxybenzyl esters and α - cyano - m - phenoxybenzyl esters of 2-haloalkyl (oxy-, thio-, sulfinyl-, or sulfonyl) phenyl-alkanoic acids are known insecticidal and acaricidal agents. These compounds and methods for their preparation are disclosed in Berkelhammer *et. al.,* U.S. Pat. Nos. 4,178,460 and 4,199,595. In both Berkelhammer *et. al.* U.S. Pat. Nos. 4,178,460 and 4,199,595, there is disclosed the conversion of certain α - alkyl - 3 (or 4) - hydroxyphenylacetic acids having the formula

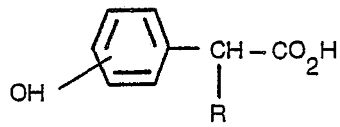

wherein R is ethyl, *n*-propyl or isopropyl to the corresponding α - alkyl - 3 (or 4) - difluoromethoxyphenylacetic acids having the formula

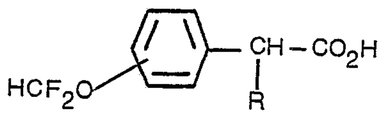

wherein R is as defined above by treatment with chlorodifluoromethane in aqueous alkali and dioxane. The α - alkyl - 3 (or 4) - difluoromethoxyphenylacetic acids thus formed are then treated with thionyl chloride, thionyl bromide, or the like, preferably in the presence of an aromatic solvent such as benzene or toluene, to yield α-alkyl (substituted phenyl)acetyl halide which is reacted with m-phenoxybenzyl alcohol or α - cyano - m - phenoxybenzyl alcohol to yield the desired m-phenoxybenzyl ester or α - cyano - m - phenoxybenzyl ester of the 2-haloalkyl(oxy-, thio-, sulfinyl- or sulfonyl)phenylalkanoic acids which are useful insecticides. In Berkelhammer *et. al.,* U.S. Pat. Nos. 4,178,460 and 4,199,595, the α - alkyl - 3 (or 4) - hydroxyphenylacetic acid intermediate is prepared by reacting the appropriate α - alkyl - 3 (or 4) - methoxyphenylacetonitrile with hydrobromic acid.

A new process for the synthesis of α - alkyl - 4 - hydroxyphenylacetic acids now has been discovered in which these materials can be prepared in a simple and straightforward manner. In this new process, 4 - (α - alkyl - α - cyano - methyl) - 2,6 - di - substituted phenols are produced in a novel synthesis reaction and are used as intermediates in a reaction sequence in which α - alkyl - 4 - hydroxyphenylacetic acids are likewise produced and used as reaction intermediates.

Methods are known for preparing 4 - (α - alkyl - α - cyano - methyl)2,6 - di - substituted phenols. For example, the preparation of 4 - (α - alkyl - α - cyano - methyl)2,6 - di - substituted phenol by reacting α - alkyl - 4 - hydroxy - 3,5 - di - *tertiary* - butylbenzyl halides with sodium cyanide is reported by A. A. Volod'kin *et. al., Iz. Akad. Nauk. SSSR. Ser. Khim,* 1966, 1031. Also, the preparation of 4 - (α - alkyl - α - cyano - methyl)2,6 - di - substituted phenol by the electrochemical reduction of the corresponding 2,6-di-substituted methylenequinones is reported by L. I. Kudinova, *et. al., Iz. Akad. Nauk. SSSR. Ser. Khim.,* 1978, 1313.

The synthesis of *o*- and *p*-hydroxy substituted phenylacetonitriles also is known and is reported in the literature. See, for example, *Journal of Organic Chemistry,* Vol. 41, No. 14, 2502 (1976).

This invention thus involves in one embodiment the discovery that 4 - (α - alkyl - α - cyano - methyl)2,6 - di - substituted phenol can be readily prepared in good yield with high selectivity by reacting a 2,6-di-substituted phenol with an aliphatic aldehyde selected from formaldehyde, acetaldehyde, propionaldehyde or butyraldehyde and an alkali metal cyanide or an alkaline earth metal cyanide in a

suitable reaction solvent to form the corresponding 4 - (α - alkyl - α - cyano - methyl)2,6 - di - substituted phenol.

In another embodiment of this invention, α - alkyl - 4 - hydroxyphenylacetic acid is produced by (1) forming a 4 - (α - alkyl - α - cyano - methyl)2,6 - di - substituted phenol in the above manner, (2) dealkylating the substituent groups ortho to the hydroxyl group from the 4 - (α - alkyl - α - cyano - methyl)2,6 - di - substituted phenol to produce a reaction product containing a substantial amount of the corresponding 4 - (α - alkyl - α - cyano - methyl) - phenol, and (3) thereafter converting the 4 - (α - alkyl - α - cyano - methyl) - phenol to the corresponding α - alkyl - 4 - hydroxyphenylacetic acid by hydrolysis.

The phenols which are used as starting materials in the process of the invention are phenols having the general formula

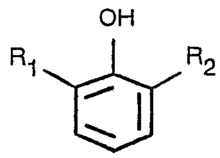

wherein each R is the same or different monovalent substituent selected from the group consisting of alkyl, aralkyl and cyclic alkyl radicals. These phenols are reacted in a liquid phase with an aldehyde selected from formaldehyde, acetaldehyde, propionaldehyde, butyraldehyde or isobutyraldehyde and an alkali metal or an alkaline earth metal cyanide.

Typical examples of alkyl, aralkyl and cyclic alkyl radicals which $R_1$ and $R_2$ may include any of the above radicals having any number of carbon atoms as long as these substituents do not interfere either with the formation of the desired 4 - (α - alkyl - α - cyano - methyl)2,6 - di - substituted phenol or with the subsequent dealkylation of the 4 - (α - alkyl - α - cyano - methyl)2,6 - di - substituted phenol to produce the corresponding 4 - (α - alkyl - α - cyano - methyl)phenol. These may include, for example, from 1 to 40 or more carbon atoms and the alkyl radicals may include primary, secondary or tertiary alkyl groups and cycloalkyl groups. Since the most readily available of the substituted phenols are those phenols having substituents of from 1 to about 8 carbon atoms they are preferred. Examples of typical substituents include methyl, ethyl, propyl, isopropyl, the isomeric butyl radicals (i.e., *n*-butyl, isobutyl, cyclobutyl, *t*-butyl, etc.), the isomeric amyl radicals, the isomeric hexyl radicals, the isomeric decyl radicals, the isomeric hexadecyl radicals, the isomeric eicosyl radicals, the isomeric tricosyl radicals, the isomeric triacontyl radicals, etc. The alkyl radicals may be substituted with aryl, preferably, monocyclic aryl radicals, or cycloalkyl radicals, for example, benzyl, phenylethyl, cyclohexylethyl, naphthylethyl, etc. Examples of aryl radicals are phenyl, tolyl, xylyl, biphenylyl, naphthyl, methylnaphthyl, ethylphenyl, cyclohexophenyl, etc. Because the phenols in which the R substituents are methyl, ethyl, propyl, butyl, *sec*-butyl, isopropyl, *t*-butyl, amyl, *sec*-amyl, *t*-amyl, hexyl, heptyl, octyl, etc., or phenyl are either readily available commercially or easily made and are ideally suited for the process, the most preferred substituents are where $R_1$ and $R_2$ are a lower alkyl group (i.e., from 1 to about 8 carbon atoms) or phenyl.

Examples of phenols having the R substituents groups noted above which are preferred starting materials include 2,6 - di - methylphenol, 2,6 - di - *sec* - butylphenol, 2,6-diisopropylphenol, 2,6 - di - *sec* - octylphenol, 2,6 - di - (α - methylbenzyl)phenol, 2 - amyl - 6 - methylphenol, 2,6 - dibenzylphenol, 2 - methyl - 6 - benzylphenol and the like. A particularly preferred phenol reactant for use in the practice of the process is 2,6 - di - *tert* - butylphenol.

The aldehyde reactant used in the process is an aldehyde having a single aldehyde radical and is selected from either formaldehyde, acetaldehyde, propionaldehyde, butyraldehyde or iso-butyraldehyde.

The alkali earth and alkaline earth metal cyanide reactants used in the present process may include sodium cyanide, potassium cyanide, lithium cyanide, magnesium cyanide and calcium cyanide. Sodium cyanide is a preferred cyanide reactant.

The reaction is carried out in the liquid phase which is provided by using a solvent which is inert under the reaction conditions. That is, the reaction is carried out in the presence of a solvent which does not enter into the reaction. Preferred solvents are aprotic solvents which include ethers such as di-ethyl ether, dibutyl ether, 1-ethoxyhexane, tetrahydrofuran, 1,4-dioxane, 1,3-dioxolane, diglyme, 1,2-diethoxyethane and tertiary amines such as pyridine, N-ethylpiperidine, triethylamine, tributylamine, N,N - diphenyl - N - methyl amine, N,N-dimethylalanine, etc. Especially preferred solvents are dipolar aprotic solvents such as dimethyl sulfoxide, N,N-dimethylformamide, N,N-dimethylacetamide, dimethyl sulfone, tetramethylene sulfone, N-methylpyrrolidinone, acetonitrile and like materials. Other solvents which are inert under the reaction conditions may be used: for example, low boiling hydrocarbons, halogenated hydrocarbons, examples of which are benzene, toluene, tetrachloroethane, the chlorinated benzenes, the chlorinated toluenes, etc. Additionally, lower alkanols having up to about 6 carbon atoms also may be used. These include methanol, ethanol, *n*-propanol, isopropyl alcohol, *n*-butanol, *sec*-butyl alcohol, *tert*-butyl alcohol, *n*-pentanol, isopentyl alcohol, *n*-hexanol and isohexyl alcohol. In addition, a small amount of water may be added to the reaction mixture to facilitate the solubilization of the cyanide-containing reactant in the mixture.

The reaction is readily conducted by placing the 2,6-di-substituted phenol and the other reaction

mixture components in a reaction vessel having agitation means. The process is preferably conducted in a substantially anhydrous reaction system, and accordingly, the components of the reaction system should be brought together and maintained under a substantially dry, inert atmosphere. Thus, while it is possible to conduct this process in the presence of air or moisture, as when water is added to the reaction mixture, it is desirable to maintain the reaction system under an atmosphere of dry nitrogen or the like.

The mode of addition is not particularly critical. Accordingly, it is convenient to add the phenol reactant to a mixture of the other materials, add the aldehyde reactant to a mixture of the other materials, add the cyanide reactant to a mixture of the other materials, introduce all ingredients simultaneously into the reaction zone or the like. The process should be carried out for a time sufficient to convert substantially all of the phenol reactant to the corresponding 4 - (α - alkyl - α - cyano - methyl)2,6 - di - substituted phenol intermediate. In general, the length of time for optimum yield depends primarily on the reaction temperature and the particular solvent used in the reaction. However, reaction ordinarily proceeds very rapidly and thus, long reaction times are not required. The reaction can be completed in the matter of minutes or at most a few hours at the reaction conditions.

Although the reaction will proceed at a very slow rate at ambient temperatures, it is convenient to conduct the reaction at an elevated temperature of at least about 50°C up to the decomposition temperature of any of the reactants or the products. Ambient atmospheric pressure can be used or pressures lower or higher than ambient pressures can be used. However, there is no advantage to using less than ambient pressure. Higher than ambient pressure conditions are usually used if temperatures higher than the boiling point at atmospheric conditions of the reaction mixture are being used. However, by proper choice of the solvent to form the liquid phase desired, temperatures can be reached within the range of about 50°C up to the reflux temperature of the reaction mixture at ambient atmospheric conditions which give a suitable reaction rate.

Conversion of the 2,6-di-substituted phenol reactant to the corresponding 4 - (α - alkyl - α - cyano - methyl)2,6 - di - substituted phenol in accordance with the practice of the invention results in substantially very little by-product formation, such as unreacted phenol, 4 - (α - alkenyl - α - methyl)2,6 - di - substituted phenol and bis - (3,5 - di - *t* - butyl - 4 - hydroxyphenyl)alkylmethane. Recovery of the product is achieved by conventional means such as evaporation and water wash or extraction with a suitable organic solvent.

For best results, it is desirable to employ an excess of both the aldehyde and cyanide reactants relative to the 2,6-di-substituted phenol reactant. Normally, the reaction system will contain at least one molar equivalent of aldehyde and one molar equivalent of cyanide per mole of phenol reactant and preferably the molar ratio of the aldehyde and the cyanide to the phenol is 2 or more.

In general, any of the various dealkylation procedures using conditions and catalysts known in the art for causing dealkylation may be used in removing the substituent groups ortho to the hydroxyl group from the 4 - (α - alkyl - α - cyano - methyl)2,6 - di - substituted phenol to produce a reaction product containing a substantial amount of the corresponding 4 - (α - alkyl - α - cyano - methyl)phenol intermediate providing they do not interfere with the course of the reaction. Preferably, dealkylation is achieved in high yield at elevated temperatures using an aluminum phenoxide or a Lewis acid catalyst in the presence of an aromatic or substituted aromatic compound. The conditions used for such dealkylations are well known and are reported in the literature. See, for example, *Journal of Organic Chemistry*, Vol. 34, 1160 (1969) and references cited therein, all disclosures of which are incorporated herein by reference.

The dealkylation process most conveniently employed comprises heating the 2,6-di-substituted phenol at an elevated temperature below the decomposition temperature of the desired 4 - (α - alkyl - α - cyano - methyl)phenol intermediate product, such as from about 60°C to 250°C in the presence of a dealkylation catalyst and an aromatic hydrocarbon or a substituted aromatic hydrocarbon, such as, for example, benzene, toluene, xylene and the like. Although it is not a requirement of the dealkylation process, the reaction can be carried out under an inert, non-reactive atmosphere, such as nitrogen, if desired.

In the reaction, the aromatic compound serves both as a solvent for the reaction and as an acceptor for the substituent groups ortho to the hydroxyl group in the 4 - (α - alkyl - α - cyano - methyl)2,6 - di - substituted phenol reactants which are dealkylated in a transalkylation process. Dealkylation results in the formation of substituted aromatic by-products, such as, for example, a mixture of ortho- and para-*tertiary*-butyl toluene when toluene is employed as the aromatic compound in the reaction from which the desired 4 - (α - alkyl - α - cyano - methyl)phenol intermediate product can be separated and recovered using well-known techniques such as distillation, fractional distillation, crystallization or extraction techniques, etc. It is not necessary, however, to first recover the desired intermediate phenol product from the reaction mixture prior to subsequent hydrolysis of the intermediate to the corresponding acid. For best results, it is desirable to employ an excess of aromatic or substituted aromatic compound relative to the di-substituted phenol reactant. Normally, the reaction system will contain at least 2 molar equivalents of aromatic reactant per mole of alkylated phenol reactant and preferably the molar ratio of the aromatic reactant to the alkylated phenol reactant is more than 2.

Aromatic hydrocarbons or substituted aromatic hydrocarbons which may be used in the dealkylation reaction include benzene, toluene, ethylbenzene, xylene, trimethylbenzene, tetrahydronaphthylene, isobutylbenzene, phenols (e.g., phenol, cresol, o-isopropylphenol, 4-hydroxyanisole (mono-, di-, and

tribromophenol, etc.), halobenzenes (e.g., mono-, di-, and triflurobenzenes, chlorobenzenes, bromobenzenes, chlorobromobenzenes), aromatic ethers (e.g., anisole, diphenylether, etc.), and the like.

Dealkylation of the substituted phenol in accordance with the invention is conducted, for example, by charging to a suitable reaction vessel the substituted phenol of choice, the solvent and the dealkylation catalyst, optionally under a blanket of nitrogen, and then heating to a temperature below the decomposition temperature of the desired 4 - (α - alkyl - α - cyano - methyl)phenol intermediate product, but high enough to effect dealkylation of the substituted phenol.

As pointed out hereinabove, the dealkylation reaction can be conducted over a wide temperature range below the decomposition temperature of the desired dealkylated product. While the reaction will proceed at ambient temperatures at a very slow rate, in general, dealkylation is carried out at a temperature range of from about 60°C to about 250°C and will vary within this range depending upon the solvent of choice.

In general, dealkylation is carried out at atmospheric pressure although pressures above atmospheric pressure can be used if desired.

The dealkylation reaction should be carried out for a time sufficient to convert substantially all of the substituted phenol starting material to the desired dealkylated phenol intermediate product. The length of time required to obtain substantially complete dealkylation of the substituted phenol will depend primarily upon the operating temperature and the particular substituted phenol used in the reaction.

A wide variety of catalysts known in the art for causing dealkylation may be used in the practice of the process. For example, dealkylation catalysts such as phenoxy derivatives of such elements as aluminum, magnesium, iron zinc, phosphorus, zirconium, titanium, bismuth, tin, etc., where the phenoxy moiety may be the phenoxy radical itself, the cresoxy radical, the xyloxy radical, etc. Also, Lewis acids, preferably aluminum chloride, zinc chloride, etc., which are predominantly para-directing catalysts when used as alkylation catalysts may be used for the dealkylation reaction. A most preferred dealkylation catalyst is aluminum chloride.

The amount of catalyst used is an amount sufficient to promote dealkylation of the substituted phenol reactant. While an amount as little as 0.1 mole percent up to amounts of about 20 mole percent based on the weight of the di-substituted phenol reactant can be used, for best results it is desirable to employ an even greater amount of catalyst up to, for example, 120 mole percent.

A variety of well-known hydrolysis procedures can be used for converting the 4 - (α - alkyl - α - cyano - methyl)phenol to the corresponding α - alkyl - 4 - hydroxyphenylacetic acid. The hydrolysis can be performed in the presence of water and a suitable polar organic solvent such as low-molecular weight alcohols (e.g., methanol or ethanol), 1,4-dioxane, acetone, low-molecular weight carboxylic acids (e.g., acetic acid or propionic aci), N-methylpyrrolidinone, dimethylsulfoxide or the like.

While hydrolysis may be performed in a neutral system or an acidic system, basic hydrolysis is preferred. The reagent of choice is aqueous sodium hydroxide. Reaction temperatures will usually fall between 0°C and the boiling point of the reaction medium. However, temperatures above the boiling point of the reaction medium can be utilized at elevated pressures to increase the rate of hydrolysis, if desired. These and other details of the hydrolysis reaction can be found in the literature—see, for example, March, *Advanced Organic Chemistry*, (McGraw-Hill, New York, 1977), pp. 809—10 and references cited therein, all disclosures of which are incorporated herein by reference.

The practice of this invention will be still further apparent by the following illustrative examples.

Example 1

Preparation of (α-cyano-methyl)2,6-di-tertiary-butyl phenol

2,6-di-tertiary-butyl phenol (2.06 g.; 10 mmoles), sodium cyanide (1.47 g; 30 mmoles), paraformaldehyde (0.72 g.; 24 mmoles) and dimethylformamide (8 ml) were charged to a 180 ml. Fischer-Porter tube and pressurized to $8.61 \times 10^5$ Pa of manometric pressure (125 psig) with nitrogen and heated to 140°C (oil bath temperature) for 9 hours. The resultant reaction mixture was allowed to cool to ambient temperature and the mixture was added to water, extracted with diethyl ether, the ether layer was dried ($MgSO_4$), filtered and the ether removed in a rotary evaporator to give a 56.4% yield of 4 - (α - cyano - methyl)2,6 - di - *tertiary* - butyl phenol as characterized by VPC.

Example 2

Preparation of 4-(α-isopropyl-α-cyano-methyl)2,6-di-tertiary-butyl phenol

2,6-di-*tertiary*-butyl phenol (21.42 g.; 103.8 mmoles), sodium cyanide (15.29 g.; 312 mmoles), isobutyraldehyde (17.99 g; 250 mmoles) and dimethyl formamide (83 ml) were charged to a 180 ml Fischer-Porter tube and pressurized to $8.61 \times 10^5$ Pa of manometric pressure (125 psig) with nitrogen and heated to 140°C (oil bath temperature) for 9 hours. The resultant reaction mixture was allowed to cool to ambient temperature and the mixture was poured into ~150 ml of water, and extracted with diethyl ether. The ether layer was dried ($MgSO_4$), filtered and the ether removed in a rotary evaporator. The residue was dissolved in ~30 ml of ethanol and precipitated by the slow addition of ice to yield 27.05 g (90.7%) of 4 - (α - isopropyl - α - cyano - methyl)2,6 - di - *tertiary* - butyl phenol.

In a manner similar to Example 2 above, a number of experiments were carried out varying the temperature, reaction time, pressure, and ratio of reactants. The results were analyzed by vapor phase chromatography with internal standards and are shown in Table I.

TABLE I

Preparation of 4-(α-isopropyl-α-cyano-methyl)2,6-di-tertiary-butyl phenol

| Experiment No. | 2,6-di-*tert*-butyl phenol (mmoles) | Isobutyr-aldehyde (mmoles) | NaCN (mmoles) | Solvent (ml) | Temp. (°C) | Manometric pressure ($10^5$ Pa) | Time (hr) | % Yield |
|---|---|---|---|---|---|---|---|---|
| 3 | 10 | 24 | 30 | DMF-8 ml | 95 | 8.61 | 7 | 58.5 |
| 4 | 10 | 24 | 30 | EtOH-8 ml | 140 | 8.61 | 7 | 47.0 |
| 5 | 10 | 24 | 30 | DMF-8 ml | 135 | 8.61 | 7 | 78 |
| 6 | 80 | 192 | 240 | DMF-64 ml | 140 | 8.61 | 9 | 86 |
| 7 | 10 | 24 | 30 | toluene-8 ml | 135 | 8.61 | 5 | trace |
| 8 | 103.8 | 207.6 | 207.6 | DMF-83 ml | 140 | 8.61 | 9 | 94.5 |
| 9 | 103.8 | 250 | 114.1 | DMF-83 ml | 140 | 8.61 | 9 | 54.0 |
| 10 | 103.8 | 200 | 119.4 | DMF-83 ml | 140 | 8.61 | 9 | 84.0 |
| 11 | 10 | 24 | 30 | isopropyl alc.-8 ml | 95 | 8.61 | 7 | 14.2 |
| 12 | 10 | 12 | 30 | EtOH-8 ml | 95 | 8.61 | 2.5 | 66.4 |
| 13 | 103 | 250 | 207 | DMF-83 ml | 140 | 6.89 | 9 | 92 |
| 14 | 103 | 250 | 207 | DMF-83 ml/5% $H_2O$ | 140 | 6.89 | 4 | 75 |
| 15 | 100 | 200 | 125 | DMF-83 ml/5% $H_2O$ | 140 | 6.89 | 9 | 71 |
| 16 | 103.8 | 250 | 207 | DMF-83 ml/5% $H_2O$ | 140 | 5.51 | 9 | 83.1 |
| 17 | 103.8 | 250 | 207.6 | MeOH-83 ml | 140 | 7.24 | 5.5 | 91.7 |
| 18 | 103.8 | 175 | 155.7 | MeOH-83 ml | 140 | 6.89 | 3 | 87 |
| 19 | 100 | 175 | 150 | MeOH-85 ml | 140 | 5.51 | 3 | 95 |
| 20 | 100 | 150 | 125 | MeOH-85 ml | 140 | 5.51 | 3 | 82.4 |

Example 21

Preparation of 4-(α-isopropyl-α-cyano-methyl)phenol

A solution of 4 - (α - isopropyl - α - cyano - methyl)2,6 - di - *tertiary* - butyl phenol (30.37 g; 106.2 mmoles) and 150 ml toluene was charged to a reactor equipped with a stirrer, thermometer and reflux condenser. Aluminum chloride (17 g; 127.5 mmoles) was added to the reactor vessel in 3 or 4 increments while vigorous agitation was maintained. An exotherm was observed which raised the reaction temperature by ~20°C. After the aluminum chloride addition was complete, the solution was heated to 95°C for 5 hours under nitrogen. The reaction mixture was then cooled to ambient temperature, washed twice with water to remove aluminum salts formed during the reaction and the solvent and tertiary-butyl-toluene by-product was removed under reduced pressure to yield 20.1 g (95.3%) of product determined by VPC (internal standard) as 4 - (α - isopropyl - α - cyano - methyl)phenol.

In a manner similar to Example 21 above, a number of experiments were carried out varying the temperature, reaction time, ratio of reactants and catalysts. The results were analyzed by vapor phase chromatography with internal standards (unless otherwise indicated) and are shown in Table II.

TABLE II
Preparation of 4-(α-isopropyl-α-cyano-methyl)phenol

| Experiment No. | 4-(α-isopropyl-α-cyano-2,6-di-*tertiary*-butyl)phenol (g) | Catalyst (g) | Solvent (ml) | Temp. (°C) | Time (hr) | % Yield |
|---|---|---|---|---|---|---|
| 22 | 4.80 | DEAC*—0.22 | — | 160<br>175 | 3<br>2 | 36.3—External standard |
| 23 | 4.80 | DEAC—0.22 | — | 180—185 | 6 | 56.4—External standard |
| 24 | 5.92 | DEAC—0.24 | — | 195—200 | 6 | 82.5—External standard |
| 25 | 6.5 | $AlCl_3$—0.48 | — | 175—180 | 4 | 64.8—External standard |
| 26 | 6.5 | $AlCl_3$—0.47 | — | 195—200 | 5 | 67.6—External standard |
| 27 | 7.0 | DEAC—0.20 | — | 195—200 | 5 | 50.4—External standard |
| 28 | 6.8 | DEAC—0.48 | — | 200 | 5 | 55.0—External standard |
| 29 | 4.0 | TEA**—0.29 in toluene | — | 180 | 5 | trace |
| 30 | 4.26 | DEAC—0.29 | — | 180 | 5 | 63.2 |
| 31 | 3.75 | $AlCl_3$—0.30 | — | 180 | 5 | 68.0 |
| 32 | 5.04 | DEAC—0.30 in phenol | — | 195—200 | 5 | mostly starting material |
| 33 | 7.65 | $AlCl_3$—1.50 | — | 170 | 5 | 55.9 |
| 34 | 6.01 | $AlCl_3$—0.69 | — | 170 | 5 | 63.8 |
| 35 | 6.01 | $AlCl_3$—0.69 | toluene—4.5 | 140 | 5 | 14.7 |
| 36 | 4.54 | DEAC—0.20 | — | 170 | 5 | 38.4 |
| 37 | 7.67 | $AlCl_3$—0.60 | — | 170—175 | 5 | 45.4 |
| 38 | 6.86 | $AlCl_3$—4.00 | toluene—50 | 80—85 | 5 | 91.2 |
| 39 | 6.86 | $AlCl_3$—4.00 | toluene—50 | 100—105 | 5 | 94.2 |

TABLE II (contd.)
Preparation of 4-(α-isopropyl-α-cyano-methyl)phenol

| Experiment No. | 4-(α-isopropyl-α-cyano-2,6-di-*tertiary*-butyl)phenol (g) | Catalyst (g) | Solvent (ml) | Temp. (°C) | Time (hr) | % Yield |
|---|---|---|---|---|---|---|
| 40 | 8.11 | $AlCl_3$—4.45 | toluene—60 | 90 | 5 | 92.3*** |
| 41 | 24 | $AlCl_3$—11.40 | toluene—100 | 95—100 | 4 | 93.8 |
| 42 | 10 | $AlCl_3$—5.60 | toluene—50 | 95 | 5 | 88.1 |
| 43 | 10 | $AlCl_3$—5.60 | toluene—50+$H_2O$ (.03 g) | 95 | 5 | 81.8 |
| 44 | 8.9 | $AlCl_3$—4.96 | toluene—45 | 90 | 5 | 100 |
| 45 | 9.3 | $AlCl_3$—5.20+$CH_3NO_2$****—2.38 | toluene—50 | 90 | 6 | 85.1 |
| 46 | 12.87 | $AlCl_3$—7.20 | toluene—65 | 90—95 | 4 | 90.9 |
| 47 | 28.6 | $AlCl_3$—19.34 | toluene—117 | 95 | 4 | 84.6 |

*DEAC=diethyl aluminum chloride
**triethyl aluminum
***isolated yield
****p-nitromethane

Example 48

Preparation of α-isopropyl-4-hydroxyphenylacetic acid

4-(α-isopropyl-α-cyano-methyl)phenol (12.88 g; 74 mmoles) was charged to a 30 ml stainless steel autoclave along with 17.76 g NaOH and 120 ml water. The solution was heated at 130°C for 6 hours with vigorous stirring while maintaining a pressure of between about $2.41 \times 10^5$ and $2.76 \times 10^5$ Pa of manometric pressure (35 and 40 psig). After 6 hours, the reaction vessel was cooled to ambient temperature, the reaction mixture was discharged into a separatory funnel, and the pressure vessel was washed with 30 ml of water which was added to the reaction mixture. The resultant mixture was washed with methylene chloride to remove residual *tert*-butyl toluene, cooled to ~10°C and acidified to a pH between 2 and 3 with concentrated hydrochloric acid. The product was separated by filtration, washed with water and dried under pressure (20 mm Hg/60°C) to give 14.29 g (96.0% yield) of α - isopropyl - 4 - hydroxyphenyl acetic acid as characterized by gas chromatography.

In a manner similar to Example 48 above, a number of experiments were carried out varying the temperature, reaction time, pressure and ratio of reactants. The results were analyzed by HPLC using external standards and are shown in Table III.

TABLE III
Preparation of α-isopropyl-4-hydroxyphenylacetic acid

| Experiment No. | 4-(α-isopropyl-α-cyano-methyl)-phenol (g) | Catalyst (g) | Solvent (ml) | Temp. (°C) | Manometric pressure ($10^5$ Pa) | Time (hr) | % Yield |
|---|---|---|---|---|---|---|---|
| 49 | 1.152 | NaOH— 1.39 | $H_2O$— 10 | reflux | ambient | 17 | 82.3 |
| 50 | 1.29 | NaOH— 1.48 | $H_2O$— 10 | reflux | ambient | 17 | 100 |
| 51 | 9.77 | NaOH—13.48 | $H_2O$—100 | 130° | 2.41 | 6 | 85 |
| 52 | 10.89 | NaOH—15.6 | $H_2O$—100 | 130° | 2.41 | 6 | 86.6 |
| 53 | 14.27 | NaOH—19.7 | $H_2O$—110 | 130° | 2.41 | 5 | 87.5 |

**Claims**

1. A process for the preparation of 4 - (α - alkyl - α - cyano - methyl)2,6 - di - substituted phenol having the formula

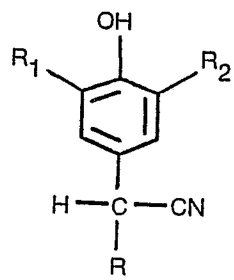

wherein R is hydrogen, methyl, ethyl, *n*-propyl or isopropyl and $R_1$ and $R_2$ are the same or different monovalent substituent selected from the group consisting of alkyl, aralkyl and cyclic alkyl radicals which comprises reacting a di-substituted phenol having the formula

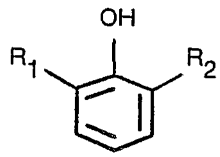

wherein $R_1$ and $R_2$ are as defined above with an aliphatic aldehyde selected from formaldehyde, acetaldehyde, propionaldehyde, butyraldehyde or isobutyraldehyde and an alkali metal or an alkaline earth metal cyanide in a suitable reaction solvent to form said 4 - (α - alkyl - α - cyano - methyl)2,6 - di - substituted phenol.

2. The process of Claim 1 wherein $R_1$ and $R_2$ are the same or different monovalent substituent selected from the group consisting of alkyl, aralkyl and cyclic alkyl radicals containing from 1 to 40 carbon atoms.

3. The process of Claim 1 wherein the alkali metal cyanide is sodium cyanide.

4. The process of Claim 1 wherein the solvent is a dipolar aprotic solvent.

5. The process of Claim 4 wherein the solvent is selected from the group consisting of dimethyl sulfoxide, N,N-dimethylformamide, N,N-dimethylacetamide, dimethyl sulfone, tetramethylene sulfone, N-methylpyrrolidinone and acetonitrile.

6. The process of Claim 4 wherein the solvent is selected from the group consisting of a lower alkanol having from 1 to about 6 carbon atoms.

7. The process of Claim 1 wherein the reaction is carried out at an elevated temperature.

8. The process of Claim 7 wherein the process is carried out at a temperature of at least 50°C.

9. The use of the 4 - (α - alkyl - α - cyano - methyl)2,6 - di - substituted phenols as defined in Claim 1 for preparing α - alkyl - 4 - hydroxyphenyl - acetic acid having the formula

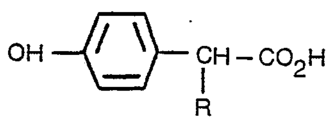

(i) dealkylating the substituent groups ortho to the hydroxyl group from said 4 - (α - alkyl - α - cyano - methyl)2,6 - di - substituted phenol to form the corresponding 4 - (α - alkyl - α - cyano - methyl)phenol, and (ii) converting said 4 - (α - alkyl - α - cyano - methyl)phenol by hydrolysis to the corresponding α - alkyl - 4 - hydroxyphenylacetic acid.

10. The use in Claim 9 wherein dealkylation is effected by heating said 4 - (α - alkyl - α - cyano - methyl)2,6 - di - substituted phenol at an elevated temperature in the presence of a dealkylation catalyst and an aromatic hydrocarbon, preferably said dealkylation catalyst being selected from a phenoxy derivative of aluminum, magnesium, iron, zinc, phosphorus, zirconium, titanium, bismuth or tin and said aromatic hydrocarbon being selected from the group consisting of benzene, toluene, ethylbenzene, xylene, trimethyl benzene, tetrahydronaphthylene, isobutylbenzene, phenol, cresol, o-isopropylphenol, 4-hydroxyanisole, halobenzenes and aromatic ethers.

**Patentansprüche**

1. Verfahren zur Herstellung von 2,6-disubstituierten 4 - (α - Alkyl - α - cyanmethyl) - phenolen der Formel

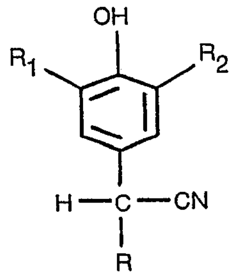

in der R Wasserstoff, Methyl, Ethyl, n-Propyl oder Isopropyl bedeutet und $R_1$ und $R_2$ gleiche oder verschiedene monovalente Substituenten sind, ausgewählt aus der Gruppe, bestehend aus Alkyl, Aralkyl und cyclischen Alkylresten, indem man ein disubstituiertes Phenol der Formel

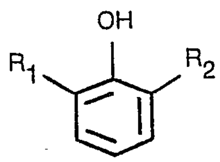

in der $R_1$ und $R_2$ die vorhin angeführte Bedeutung aufweisen, mit einem aliphatischen Aldehyd, ausgewählt aus Formaldehyde, Acetaldehyd, Propionaldehyd, Butyraldehyd oder Isobutyraldehyd und einem Alkalimetall oder einem Cyanid eines Erdalkalimetalls in einem geeigneten Reaktionslösungsmittel umsetzt, um das 2,6-disubstituierte 4 - (α - Alkyl - α - cyanmethyl) - phenol zu bilden.

2. Verfahren nach Anspruch 1, dadurch gekennzeichnet, daß $R_1$ und $R_2$ einen gleichen oder einen verschiedenen monovalenten Substituenten bedeuten, ausgewählt aus der Gruppe, bestehend aus Alkyl, Aralkyl und cyclischen Alkylresten, die 1 bis 40 Kohlenstoffatome aufweisen.

3. Verfahren nach Anspruch 1, dadurch gekennzeichnet, daß das Alkalimetallcyanid Natriumcyanid ist.

4. Verfahren nach Anspruch 1, dadurch gekennzeichnet, daß das Lösungsmittel ein dipolares, aprotisches Lösungsmittel ist.

5. Verfahren nach Anspruch 4, dadurch gekennzeichnet, daß das Lösungsmittel aus der Gruppe, bestehend aus Dimethylsulfoxid, N,N-Dimethylformamid, N,N-Dimethylacetamid, Dimethylsulfon, Tetramethylsulfon, N-Methylpyrrolidon und Acetonitril, ausgewählt ist.

6. Verfahren nach Anspruch 4, dadurch gekennzeichnet, daß das Lösungsmittel aus der Gruppe, bestehend aus einem niedrigen Alkanol mit 1 bis 6 Kohlenstoffatomen, ausgewählt ist.

7. Verfahren nach Anspruch 1, dadurch gekennzeichnet, daß man die Reaktion bei erhöhter Temperatur durchführt.

8. Verfahren nach Anspruch 7, dadurch gekennzeichnet, daß man das Verfahren bei einer Temperatur von mindestens 50°C durchführt.

9. Verwendung der 2,6-disubstituierten 4 - (α - Alkyl - α - cyanmethyl) - phenole nach Anspruch 1 zur Herstellung von α - Alkyl - 4 - hydroxyphenylessigsäure der Formel

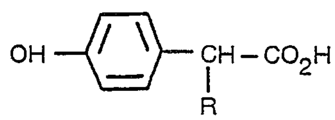

durch (i) Dealkylierung der Substituentengruppen in ortho-Stellung zur Hydroxylgruppe im 2,6-disubstituierten 4 - (α - Alkyl - α - cyanmethyl) - phenol zur Bildung des entsprechenden 4 - (α - Alkyl - α - cyanmethyl) - phenols, und (ii) durch Überführen des 4 - (α - Alkyl - α - cyanmethyl) - phenols mittels Hydrolyse in die entsprechende α - Alkyl - 4 - hydroxyphenyl-essigsäure.

10. Verwendung nach Anspruch 9, dadurch gekennzeichnet, daß die Dealkylierung des 2,6-disubstituierten 4 - (α - Alkyl - α - cyanmethyl) - phenols durch Erwärmung bei erhöhter Temperatur in Gegenwart eines Dealkylierungskatalysators und eines aromatischen Kohlenwasserstoffes erfolgt, wobei man vorzugsweise den Dealkylierungskatalysator aus einem Phenoxy-Derivat von Aluminium, Magnesium, Eisen, Zink, Phosphor, Circonium, Titan, Wismuth oder Zinn auswählt und den aromatischen Kohlenwasserstoff aus der Gruppe, bestehend aus Benzol, Toluol, Ethylbenzol, Xylol, Tri-methylbenzol, Tetrahydronaphthylen, Isobutylbenzol, Phenol, Cresol, o-Isopropylphenol, 4-Hydroxyanisol, halobenzole und aromatische Äther, auswählt.

**Revendications**

1. Procédé de préparation d'un 4 - (α - alkyl - α - cyanométhyl)phénol disubstitué en positions 2,6, de formule

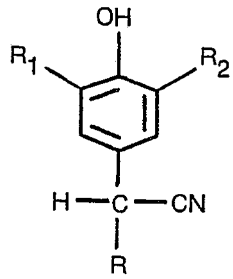

dans laquelle R désigne l'hydrogène, un groupe méthyle, éthyle, n-propyle ou isopropyle et $R_1$ et $R_2$ représentent des substituants monovalents identiques ou différents choisis dans le groupe comprenant des radicaux alkyle aralkyle et cycloalkyle, qui consiste à faire réagir un phénol disubstitué de formule

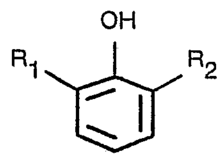

dans laquelle $R_1$ et $R_2$ sont tels que définis ci-dessus, avec un aldéhyde aliphatique choisi entre le formaldéhyde, l'acétaldéhyde, le propionaldéhyde, le butyraldéhyde et l'isobutyraldéhyde, et un cyanure de métal alcalin ou un cyanure de métal alcalino-terreux dans un solvant réactionnel convenable pour former ledit 4 - (α - alkyl - α - cyanométhyl)phénol disubstitué en positions 2,6.

2. Procédé suivant la revendication 1, dans lequel $R_1$ et $R_2$ sont des substituants monovalents identiques ou différents choisis dans le groupe comprenant des radicaux alkyle, aralkyle et cycloalkyle ayant 1 à 4 atomes de carbone.

3. Procédé suivant la revendication 1, dans lequel le cyanure de métal alcalin est le cyanure de sodium.

4. Procédé suivant la revendication 1, dans lequel le solvant est un solvant aprotique dipolaire.

5. Procédé suivant la revendication 4, dans lequel le solvant est choisi dans le groupe comprenant le diméthylsulfoxyde, le N,N-diméthylformamide, le N,N-diméthylacétamide, la diméthylsulfone, la tétraméthylènesulfone, la N-méthylpyrrolidinone et l'acétonitrile.

6. Procédé suivant la revendication 4, dans lequel le solvant est choisi dans le groupe comprenant un alcanol inférieur ayant 1 à environ 6 atomes de carbone.

7. Procédé suivant la revendication 1, dans lequel la réaction est conduite à une température élevée.

8. Procédé suivant la revendication 7, dans lequel le procédé est mis en oeuvre à une température d'au moins 50°C.

9. Utilisation des 4 - (α - alkyl - α - cyanométhyl)phénols disubstitués en positions 2,6 tels que définis dans la revendication 1 pour la préparation d'un acide α - alkyl - 4 - hydroxyphénylacétique de formule

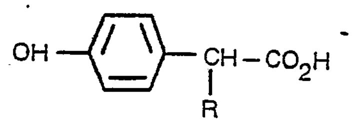

par (i) déalkylation des groupes substituants en ortho par rapport au groupe hydroxyle venant du 4 - (α - alkyl - α - cyanométhyl)phénol disubstitué en positions 2,6 pour former le 4 - (α - alkyl - α - cyanométhyl)phénol correspondant et (ii) transformation dudit 4 - (α - alkyl - α - cyanométhyl)phénol par hydrolyse en l'acide α - alkyl - 4 - hydroxyphénylacétique correspondant.

10. Utilisation suivant la revendication 9, dans laquelle la déalkylation est effectuée par chauffage dudit 4 - (α - alkyl - α - cyanométhyl)phénol disubstitué en positions 2,6 à une température élevée en présence d'un catalyseur de déalkylation et d'un hydrocarbure aromatique, ledit catalyseur de déalkylation étant avantageusement choisi entre un dérive phénoxy d'aluminium, de magnésium, de fer, de zinc, de phosphore, de zirconium, de titane, de bismuth ou d'étain et ledit hydrocarbure aromatique étant choisi dans le groupe comprenant le benzène, le toluène, l'éthylbenzène, un xylène, le triméthylbenzène, le tétrahydronaphtylène, l'isobutylbenzène, le phénol, le crésol, le o-isopropylphénol, le 4-hydroxyanisole, des halogénobenzènes et des éthers aromatiques.